# DEMANDE DE BREVET EUROPEEN

(11) **EP 1 354 596 A1**
(43) Date de publication de la demande: **22.10.2003**
(21) Numéro de dépôt: 03354032.9
(22) Date de dépôt: 14.04.2003
(51) Int. Cl.: A61K 35/78, A61P 37/08

(54) **Solution médicamenteuse comportant un extrait d'oeuf de caille et une solution d'extraits de plantes médicinales**

(30) Priorité: 15.04.2002 FR 0204702
(71) Demandeur: Betend-dit-Bon, Michel, 74940 Annecy Le Vieux (FR)
(72) Inventeur: Betend-dit-Bon, Michel, 74940 Annecy Le Vieux (FR)
(74) Mandataire: Hecké, Gérard

(57) **Abrégé**

L'invention porte sur de nouvelles solutions médicamenteuses associant l'oeuf de caille japonaise aux plantes médicinales administrées par voie orale et sublinguale dans les allergies, la pathologie générale et leur procédé de fabrication. On prépare une solution neutre d'excipient dans laquelle on incorpore des extraits liquides d'oeufs de caille japonaise et un ou des extraits fractionnés ou totaux de plantes médicinales selon la méthode de l'agitation fractionnée ou toute autre technique permettant d'obtenir lesdites solutions. Ces nouvelles solutions et l'obtention de nouvelles formes galéniques élargissent les indications de ces médicaments en médecine humaine et vétérinaire dans les maladies immuno-allergiques et en pathologie générale et sont donc un nouvel apport thérapeutique de premier choix.

## Description

### Domaine technique de l'invention

La présente invention concerne une composition médicamenteuse destinée à la médecine humaine et vétérinaire, pour administration orale, per- et sublinguale et comportant un extrait d'oeuf de caille.

### Etat de la technique

Il a été proposé, dans le document FR-A-2811898, des formes galéniques médicamenteuses homéopathiques d'allergènes imprégnés sur un support biologiquement actif. Le support biologiquement actif est formé par un homogénat d'oeuf de caille incorporé à un excipient comportant, par exemple, un produit de charge, un produit liant, un produit de désagrégation et un produit lubrifiant.

### Objet de l'invention

L'invention a pour objet une nouvelle composition médicamenteuse indiquée particulièrement en particulier dans les maladies immuno-allergiques mais également dans toutes les autres pathologies.

L'invention est plus particulièrement définie dans les revendications annexées.

### Description de modes particuliers de réalisation

La présente invention concerne plus particulièrement une composition médicamenteuse comportant une solution neutre d'excipient dans laquelle on incorpore, par exemple une solution d'homogénat d'oeufs de caille japonaise ou une solution de blanc d'oeufs de caille seul et une ou plusieurs solutions d'extraits de plantes médicinales utilisées en phytothérapie, pour administration par voie orale et per et sub-linguale, par la technique de l'agitation fractionnée ou par toute autre technique permettant d'obtenir une solution homogène et stable des principes actifs incorporés dans la solution d'excipient.

Par « solution neutre d'excipient » on entend, par exemple, une ou plusieurs préparations obtenues à partir du sorbitol à 70%, de la carboxymethylcellulose, des conservateurs comme le methyl-p-hydroxybenzoate de soude et le propyl-p-hydroxybenzoate de soude, un arôme naturel et de l'eau purifiée, dans laquelle on incorpore par agitation fractionnée des principes actifs comme par exemple une solution d'homogénat d'oeufs de caille japonaise ou une solution de blanc d'oeufs de caille seul et des solutions de plantes médicinales :
- les oeufs de cailles sont connus depuis la plus haute antiquité pour leurs vertus « médicinales » mais de manière empirique, et ajoutés à la solution neutre d'excipient ils lui confèrent des propriétés anti-protéasiques qui sont de découvertes récentes;
- les plantes médicinales elles, sont connues depuis la préhistoire et leur activité et leur efficacité ne sont plus à démontrer puisque l'on sait que la synthèse de nombreux médicaments allopathiques est faite à partir de ces plantes, le meilleur exemple étant l'aspirine.

Par « oeufs de caille » on entend par exemple l'oeuf de l'espèce animale coturnix coturnix japonica et notamment la souche B. Mina.

Par « extrait d'oeuf de caille » on entend par exemple toutes fractions ou parties biologiquement actives (l'oeuf de caille étant considéré comme un produit naturel) obtenues après différents traitements mécaniques, physiques ou chimiques, ces différents modes de traitements n'étant pas limitatifs, et qui permettent de séparer ou d'enrichir tout constituant dudit oeuf que ce soit le blanc ou le jaune ou toute autre partie dudit oeuf ; de tels extraits dudit oeuf sont en général obtenus sous forme homogène à l'état sec, par séchage par exemple, ce qui permet d'avoir une poudre ou, à l'état humide d'avoir un liquide.

Par « homogénat d'oeuf de caille » on entend un mélange aqueux de blancs et de jaunes d'oeufs de caille enrichi en blanc qui comporte par exemple et selon l'invention :
- un mélange homogène de blancs et de jaunes d'oeufs de cailles de souche B.Mina, auquel on ajoute :
   - une quantité de blancs seuls de volume égal à celui que l'on a obtenu avec le mélange précédent(jaunes et blancs) ; dans cet homogénat, la proportion de blanc par rapport au jaune est de 2 pour 1, par exemple.

Par « jaune d'oeuf de caille » on entend exclusivement le jaune séparé du blanc que l'on utilise par exemple dans des proportions contrôlées et reproductibles et dont on prélève une partie aliquote selon les modalités de fabrication. Le jaune d'oeuf de caille de souche B.Mina est caractérisé par une action liposomiale, c'est à dire qu'il sert de vecteur aux principes actifs qu'il conduit vers les sites récepteurs de l'organisme ; de plus, il possède une teneur importante en acides aminés qui favorisent l'absorption de certains oligo-éléments par l'organisme comme le zinc par exemple, ce qui augmente les défenses immunitaires; le jaune contient une faible quantité de cholestérol.

Par « blanc d'oeuf de caille » on entend exclusivement le blanc séparé du jaune que l'on utilise comme constituant actif de l'oeuf de caille dans des proportions contrôlées et reproductibles dont on prélève une partie aliquote, par exemple, selon les modalités de fabrication. Le blanc d'oeuf de caille peut être utilisé seul, selon l'invention ; il est caractérisé par plusieurs propriétés biologiques et pharmacologiques : sa propriété principale est l'action anti-protéasique très puissante liée à sa fraction ovomucoïde qui lui confère :
- une activité anti-inflammatoire non spécifique et,
- une activité anti-allergique non spécifique qui se traduit par une inhibition de la libération des médiateurs chimiques responsables des manifestations allergiques.
   Le blanc d'oeuf de caille contient également des acides aminés essentiels qui favorisent l'absorption du zinc par l'organisme ce qui augmente les défenses immunitaires. Le mélange homogène de blancs et de jaunes d'oeufs de caille et le blanc d'oeuf de caille seul induisent un état de tolérance dès qu'ils entrent en contact avec la muqueuse buccale et sublinguale, par exemple, ce qui a pour conséquence de libérer des facteurs suppresseurs comme le TGF-β₁ qui bloque la synthèse des anticorps de la classe des IgE, classe potentialisée par l'interleukine 4 (IL4) ; les IgE sont des anticorps nocifs responsables par exemple de l'inflammation allergique et participent également à l'inflammation microbienne, virale, mycosique, fungique et parasitaire.

Par « plantes médicinales », on entend toutes les plantes dont l'efficacité à été démontrée en médecine humaine et vétérinaire et qui figurent à la Pharmacopée ; après préparation pharmaceutique on peut les utiliser entières ou en partie, selon la fraction la plus pharmacologiquement active de la plante, par exemple, et selon le procédé industriel qui a été choisi pour préparer, par exemple, une poudre à solubiliser issue de la plante entière ou de l'une de ses fractions ; d'autres préparations galéniques peuvent être préparées à partir de chaque fragment des dites plantes médicinales.

Par « extrait de plantes médicinales », on entend une partie de la plante comme, par exemple, la racine, la tige, la feuille, la fleur ou le fruit ou tout autre constituant actif de ladite plante utilisée en phytothérapie.

Conformément à la présente invention, on propose une solution médicamenteuse composée d'une solution neutre d'excipient, d'une solution d'extrait d'oeuf de caille, tel que décrit plus haut (homogénat ou blanc seul) et d'une solution de plantes médicinales (extrait ou plante totale), par exemple.

Selon l'invention et particulièrement dans le traitement des maladies inflammatoires allergiques on peut utiliser, par exemple, le plantain indiqué dans le rhume des foins, la rhinite pérannuelle, la sinusite, la laryngite, la trachéite et l'asthme ; ces exemples ne sont évidemment pas limitatifs.

La feuille de plantain, par exemple et selon la présente invention, contient des substances pharmacologiquement actives comme les iridoïdes dont le principal est l'aucuboside ; le plantain est indiqué dans le traitement des affections bronchopulmonaires ; les mucilages présents ont une action adoucissante et émolliente qui facilitent l'expectoration.

Par ailleurs, grâce à sa composition, le plantain est également anti-inflammatoire et anti-allergique. Il est donc tout indiqué dans les manifestations allergiques et son action anti-inflammatoire permet de le proposer également dans les bronchites, les pharyngites et les laryngites.

Toute autre fraction ou composant d'une plante médicinale ou la totalité de ladite plante peut être incorporée à la solution d'excipient neutre contenant une solution d'extrait d'oeuf de caille.

Selon l'invention, le marrube blanc, par exemple, est une plante médicinale vivace à feuilles dentelées duveteuses et à petites fleurs blanches ; ces fleurs contiennent des lactones diterpéniques, dont la marrubiine, conférant au marrube blanc d'étonnantes propriétés sur le système respiratoire : il fluidifie les secrétions bronchiques et facilite l'expectoration, il est antitussif et calme très bien la toux rebelle des trachéites. La présence de mucilages adoucissants et anti-inflammatoires justifie son emploi dans les inflammations de la gorge ; dilatateur des bronches, il est tout indiqué dans l'asthme et son association au plantain par exemple, induit une synergie d'action étonnante qui en maximise ses effets.

Par « arôme » on entend des arômes naturels comme le citron, l'orange, la vanille, la fraise, la framboise ou la banane par exemple, les arômes cités n'étant pas limitatifs ; on ajoute un arôme au produit fini pour en améliorer l'acceptabilité, par exemple.

Il n'était pas évident pour l'homme de l'art d'utiliser une solution neutre d'excipient et d'y incorporer une solution d'oeuf de caille associée à des solutions de plantes médicinales (fractions ou plantes entières) par la technique de l'agitation fractionnée par exemple ou toutes autres techniques selon les bonnes pratiques de fabrication, afin d'obtenir une ou plusieurs solutions médicamenteuses ou toutes autres formes galéniques pharmaceutiquement acceptables pour administration orale et per et sublinguale possédant les activités anti-protéasiques de l'oeuf de caille japonaise associé aux propriétés pharmaceutiques des plantes médicinales comme le plantain et le marrube blanc par exemple.

Selon l'invention, la solution médicamenteuse obtenue potentialisée par le mélange des solutions d'oeuf de caille et d'une ou de plusieurs solutions de plantes médicinales incorporées à une solution neutre d'excipient élargit considérablement les moyens de traitements des maladies immuno-allérgiques, des inflammations respiratoires, et des affections observées en pathologie générale ; sa facilité d'emploi est remarquable chez l'enfant et chez l'adulte et la compliance au traitement est excellente.

Ces nouvelles possibilités thérapeutiques n'étaient pas prévisibles auparavant et elles sont indiquées non seulement dans les maladies immuno-allergiques comme par exemple, la rhinite perannuelle, la pollinose, la trachéite spasmodique, l'urticaire, I' oedème de Quincke, l'eczéma atopique, les allergies alimentaires (ces exemple n'étant pas limitatifs), mais aussi en pathologie générale.

D'autres formes galéniques des principes actifs décrits dans l'invention peuvent être utilisées comme, par exemple, de la poudre, des granules, des globules, des comprimés, des gélules et toutes les formes galéniques pharmaceutiquement acceptables.

### 1/Obtention d'une solution neutre d'excipient selon l'invention

Cette solution neutre va servir d'excipient et selon l'invention elle comporte par exemple :
- un produit édulcorant massif comme le sorbitol à 70%,
- un agent de suspension et viscosant comme la carboxymethylcellulose,
- des conservateurs comme :
   - le methyl-p-hydroxybenzoate de soude,
   - le propyl-p-hydroxybenzoate de soude,
- un arôme naturel, comme l'orange,
- un diluant, comme l'eau purifiée.

Un exemple de formulation selon l'invention est décrit ci-après.

### 2/Formulation de la solution neutre d'excipient selon l'invention

La solution neutre d'excipient à laquelle on va incorporer une solution d'homogénat d'oeuf de caille ou d'une solution de blanc d'oeuf de caille seul ou plusieurs plantes médicinales, est composée par exemple par :
- du sorbitol à 70 % 62,500 g
- de la carboxymethycellulose 0,500 g
- du methyl-p-hydroxybenzoate de soude 0,225 g
- du propyl-p-hydroxybenzoate de soude 0,025 g
- de l'arôme orange naturel 0,937 g
- de l'eau purifiée qsp 125 ml

Un exemple de procédé de fabrication de la solution neutre d'excipient selon l'invention est donné ci-après :

### 3/ Procédé de fabrication de la solution neutre d'excipient selon l'invention

On procède successivement aux étapes suivantes selon la méthode de l'agitation fractionnée, par exemple :
a)Sous agitation pendant 10 minutes : on dilue le methyl-p-hydroxybenzoate de soude et le propyl-p-hydroxybenzoate de soude dans environ 450 ml d'eau purifiée et on laisse reposer pendant environ 5 minutes ;
b)On reprend l'agitation pendant 10 minutes et on rajoute au mélange obtenu en a) de la carboxymethylcellulose jusqu'à sa dissolution complète et on laisse reposer pendant 5 minutes ;
c)On reprend l'agitation pendant environ 10 minutes et on rajoute au mélange obtenu en b) du sorbitol à 70% jusqu'à l'obtention d'une parfaite homogénéité ; on laisse reposer de nouveau pendant 5 minutes ;
d)On reprend l'agitation pendant 10 minutes et on rajoute de l'arôme naturel à l'orange, par exemple, et après obtention d'une solution homogène on laisse reposer pendant 5 minutes ;
e)On reprend l'agitation pendant 10 minutes et on rajoute de l'eau purifiée jusqu'à l'obtention d'un volume final d'un litre puis on laisse reposer 5 minutes ; cette quantité de solution d'un litre va servir à la préparation de 8 flacons de solution neutre d'excipient de 125 ml chacun par exemple.

Toute autre technique ou méthode permettant d'obtenir une solution homogène et stable de l'excipient neutre et des principes actifs peut être utilisée par exemple.

### 4/ Obtention d'une solution d' homogénat d'oeuf de caille selon l'invention :

L'homogénat comprend un mélange homogène de jaune et de blanc d'oeuf de caille enrichi en blanc d'oeuf de caille tel que décrit dans notre brevet EP 315 552 et nos brevets 28 11 898 et 28 135 31.

On utilise des oeufs de caille de toutes espèces et provenances, par exemple, la souche B. Mina, produits par des élevages placés sous surveillance sanitaire et vétérinaire.

Les oeufs de caille sont préalablement traités de manière à exclure de leur coquille tous germes pathogène, par exemple, par immersion dans un ou plusieurs bains de solutions antiseptiques. Les oeufs sont cassés mécaniquement et déversés dans une cuve maintenue sous agitation ou, par exemple, vidés par aspiration après perforation de la coquille.

Après avoir séparé le blanc et le jaune des coquilles, on procède à une filtration de manière à obtenir soit un mélange homogène de blancs et de jaunes enrichi en blanc (homogénat), soit des blancs d'oeufs de caille seuls qui servent à la préparation de formes médicamenteuses sans la moindre trace de jaune.

Un exemple de formulation de la solution d'homogénat mélangé à la solution neutre d'excipient est donné, ci-après.

### 5/ Formulation d'une solution d'homogenat d'oeuf de caille incorporée dans la solution neutre d'excipient selon l'invention

| | |
|---|---|
| Blancs et jaunes d'oeufs de caille | 0,205 g |
| Blanc d'oeuf de caille seul | 0,205 g |
| Sorbitol à 70% | 62,5 g |
| Carboxymethylcellulose | 0,500 g |
| Methyl-p-hydroxybenzoate de soude | 0,225 g |
| Propyl-p-hydroxybenzoate de soude | 0,025 g |
| Arôme naturel orange | 0,937 g |
| Eau purifiée | 125 ml |

Un exemple de procédé de fabrication d'une solution d'homogénat d'oeuf de caille est décrit ci-après, selon l'invention.

### 6/ Procédé de fabrication d'une solution d'homogénat d'oeuf de caille selon l'invention :

Après avoir séparé le blanc et le jaune des coquilles on procède à une filtration de manière à obtenir, sous forme liquide, des blancs et des jaunes d'oeufs de caille séparés.

La fabrication d'une solution d'homogénat d'oeufs de caille, selon l'invention, se fait à partir d'un mélange à parties égales de 10% (V/V) dans l'eau distillée de manière à avoir, par exemple, deux fois plus de blancs que de jaunes ; après battage, le mélange devient liquide et homogène, prêt à être incorporé dans la solution neutre d'excipient selon les formulations proposées mais non limitatives dans leur énoncé.

Un exemple de procédé d'obtention d'une solution de blanc d'oeuf de caille seul et décrit ci-après, selon l'invention.

### 7/ Obtention d'une solution de blanc d'oeuf de caille seul selon l'invention :

L'obtention du jaune et du blanc a été décrit en 4/.
Après avoir séparé les jaunes et les blancs des coquilles, les blancs sont filtrés et liquéfiés et servent à la préparation des solutions médicamenteuses ou toutes autres formes galéniques pharmaceutiquement acceptable.

Un exemple de formulation est décrit ci-après, étant bien entendu qu'il ne saurait être limitatif.

### 8/ Formulation d'une solution de blanc d'oeuf de caille seul incorporée dans la solution neutre d'excipient selon l'invention :

| | |
|---|---|
| Blanc d'oeuf de caille seul | 0,410 g |
| Sorbitol à 70% | 62,500 g |
| Carboxymethylcellulose | 0,500 g |
| Methyl-p-hydroxybenzoate de soude | 0,225 g |
| Propyl-p-hydroxybenzoate de soude | 0,025 g |
| Arôme naturel orange | 0,937 g |
| Eau purifiée | 125 ml |

Un exemple de procédé de fabrication d'une solution de blanc d'oeuf de caille, selon l'invention est décrit ci-après.

### 9/ Procédé de fabrication d'une solution de blanc d'oeuf de caille seul selon l'invention :

Après avoir séparé les jaunes et les blancs tels que décrits en 6/, on prépare les blancs qui seront liquéfiés.

Selon l'invention on prépare une solution à 10% (V/V) de blanc d'oeuf de caille seul dans de l'eau distillée; après battage le mélange devient liquide et prêt a être incorporé dans la solution neutre d'excipient, par exemple, selon les formulations proposées qui ne sont évidemment pas limitatives.

Un exemple d'obtention d'un principe actif d'une plante médicinale selon l'invention est décrit ci-après.

### 10/ Obtention d'un principe actif d'une plante médicinale selon l'invention

Des fabricants proposent tous les principes actifs des plantes médicinales préparés selon différentes méthodes :
- des extraits titrés de plantes sont obtenus par nébulisation ; c'est une technique qui assure une extraction et une conservation parfaite des principes actifs de la plante ; 1g de nébulisat correspond à 5g de plante déshydratée ; ces nébulisats ont des avantages :
   - ils garantissent la teneur en principes actifs ce qui permet d'avoir une très grande reproductibilité et une composition constante ;
   - ils garantissent une meilleur bio-disponibilité ce qui autorise à diminuer la dose administrée ;
- des extraits titrés par cryobroyage, technique qui permet d'obtenir une poudre totale ; l'emploi de la poudre totale a l'avantage de proposer l'ensemble des principes actifs de la plante ce qui assure la parfaite préservation et la restitution de l'ensemble des constituants garantissant ainsi une parfaite action synergique.
   Des exemples de formulations sont décrit ci-après et ne sont évidemment pas limitatifs.

### 11/ Formulation d'une solution médicamenteuse de feuille de plantain et d'une solution d'homogénat d'oeuf de caille incorporée dans une solution neutre d'excipient selon l'invention.

| | |
|---|---|
| Plantain (feuille) | 7 g |
| Blancs et jaunes d'oeuf de caille | 0,205 g |
| Blanc d'oeuf de caille seul | 0,205 g |
| Sorbitol à 70% | 62,50 g |
| Carboxymethycellulose | 0,500 g |
| Methyl-p-hydroxybenzoate de soude | 0,225 g |
| Propyl-p-hydroxybenzoate de soude | 0,025 g |
| Arôme naturel orange | 0,937 g |
| Eau purifiée | qsp 125 ml |

### 12/ Formulation d'une solution médicamenteuse de blanc d'oeuf de caille seul et d'une solution de feuille de plantain incorporée dans une solution neutre d'excipient selon l'invention

| | |
|---|---|
| Plantain (feuille) | 7 g |
| Blanc d'oeuf de caille | 0,410 g |
| Sorbitol à 70% | 62,500 g |
| Carboxymethylcellulose | 0,500 g |
| Methyl-p-hydroxybenzoate de soude | 0,225 g |
| Propyl-p-hydroxybenzoate de soude | 0,025 g |
| Arôme orange | 0,937 g |
| Eau purifiée | 125 ml |

### 13/ Formulation d'une solution médicamenteuse d'homogénat d'oeuf de caille, mélangée à des solutions de feuille de plantain et de fleur de marrube blanc dans une solution neutre d'excipient selon l'invention

| | |
|---|---|
| Plantain (feuille) | 3,500 g |
| Marrube blanc (fleur) | 3,500 g |
| Blanc et jaune d'oeuf de caille | 0,205 g |
| Blanc d' oeuf de caille seul | 0,205 g |
| Sorbitol à 70% | 62,500 g |
| Carboxymethylcellulose | 0,500 g |
| Methyl-p-hydroxybenzoate de soude | 0,225 g |
| Propyl-p-hydroxybenzoate de soude | 0,025 g |
| Arôme naturel orange | 0,937 g |
| Eau purifiée | qsp 125 ml |

### 14/ Formulation d'une solution médicamenteuse de blanc d'oeuf de caille seul associée à une solution de feuille de plantain et de fleur de marrube blanc dans une solution neutre d'excipient selon l'invention

| | |
|---|---|
| Plantain (feuille) | 3,500 g |
| Marrube de blanc (fleur) | 3,500 g |
| Sorbitol à 70% | 62,500 g |
| Carboxymethycellulose | 0,500 g |
| Methyl-p-hydroxybenzoate de soude | 0,225 g |
| Prophyl-p-hydroxybenzoate de soude | 0,025 g |
| Arôme naturel orange | 0,937 g |
| Eau purifiée | qsp 125 ml |

### 15/ Procédé de fabrication de solutions de plantes médicinales selon l'invention

La méthode proposée dans la présente invention est celle du cryobroyage mais, toutes les techniques utilisées selon les bonnes pratiques de fabrication peuvent être employées.

Cette méthode consiste à pulvériser la partie active de la plante sèche ou en la broyant à froid sous azote afin de recueillir une poudre parfaitement fine et homogène, ce qui a l'avantage de conserver tous les principes actifs comme les vitamines et les enzymes, par exemple ; grâce à sa fine granulométrie, la poudre totale va parfaitement se mélanger à la solution neutre d'excipient et à la solution d'extraits d'oeuf de caille(homogénat et blanc seul) ; après un temps d'agitation de 10 minutes par exemple, on obtient un mélange très stable.

On mélange ensuite intimement, 10 ml de solution neutre d'excipient dans 0.040 g d'homogénat d'oeuf de caille par exemple, ou dans 0.040 g de blanc seul et on agite pendant environ 10 minutes ; on laisse reposer pendant 5 minutes ; puis on renouvelle l'opération jusqu'à ce que toute la poudre, toute la solution d'extrait d'oeuf de caille et toute la solution neutre d'excipient aient été utilisées.

On obtient un volume voisin de 125 ml, par exemple, puis on homogénéise pendant une demi-heure ; on laisse ensuite reposer et on obtient ainsi une solution médicamenteuse parfaitement liquide et stable.

Il est bien évident que, selon l'invention, les exemples d'obtentions, de formulations et de procédés sont donnés à titre indicatif et ne sont pas limitatifs ni dans leur objet ni dans leur description.

## Revendications

1. Composition médicamenteuse destinée à la médecine humaine et vétérinaire, pour administration orale, per- et sublinguale et comportant un extrait d'oeuf de caille, composition **caractérisée en ce qu'**elle est constituée par un mélange homogène d'une solution neutre d'excipient, d'au moins une solution d'extraits de plantes médicinales et d'une solution de l'extrait d'oeuf de caille, ledit extrait étant constitué par un homogénat d'oeuf de caille ou par du blanc d'oeuf de caille.

2. Composition médicamenteuse selon la revendication 1, **caractérisée en ce que** la solution neutre d'excipient comporte un produit édulcorant massif, un agent de suspension et viscosant, des conservateurs, un arôme naturel et un diluant.

3. Composition médicamenteuse selon la revendication 2, **caractérisée en ce que** l'arôme naturel est choisi parmi l'orange, le citron, la vanille, la fraise, la framboise et la banane.

4. Composition médicamenteuse selon l'une quelconque des revendications 1 à 3, **caractérisée en ce que** le mélange homogène est obtenu par une technique d'agitation fractionnée.

5. Composition médicamenteuse selon l'une quelconque des revendications 1 à 4, **caractérisée en ce que** la solution de l'extrait d'oeuf de caille étant constituée par un homogénat d'oeuf de caille, l'homogénat comporte un mélange de blancs d'oeuf de caille et de jaunes d'oeuf de caille dans une proportion de 2 pour 1.

6. Composition médicamenteuse selon l'une quelconque des revendications 1 à 5, **caractérisée en ce que** les extraits de plantes médicinales sont obtenus par cryobroyage.

7. Composition médicamenteuse selon l'une quelconque des revendications 1 à 6, **caractérisée en ce que** l'extrait d'oeuf de caille est un extrait d'oeuf de caille de l'espèce coturnix coturnix japonica de la souche B.Mina.

8. Composition médicamenteuse selon l'une quelconque des revendications 1 à 7, **caractérisée en ce que** les plantes médicinales utilisées dans les extraits de plantes médicinales sont fractionnées ou entières.

9. Composition médicamenteuse selon l'une quelconque des revendications 1 à 8, **caractérisée en ce que** les extraits de plantes médicinales sont issus du plantain, du marrube blanc ou de l'association de plantain et de marrube blanc.

10. Composition médicamenteuse selon l'une quelconque des revendications 1 à 9, **caractérisée en ce que** la composition médicamenteuse étant administrée par voie per- et sublinguale, elle est sous forme de poudre, de granule, de globule, de gélule ou de comprimé.

11. Composition médicamenteuse selon l'une quelconque des revendications 1 à 10 utilisée pour la préparation de médicaments destinés à traiter les maladies immuno-allergiques.
